# FASCICULE DE BREVET EUROPEEN

(11) **EP 1 108 446 B1**
(45) Date de publication et mention de la délivrance du brevet: **04.05.2005**
(21) Numéro de dépôt: 00403481.5
(22) Date de dépôt: 12.12.2000
(51) Int. Cl.: A61N 1/368, A61N 1/365

(54) **Disposifif implantable actif de type multisite, comportant des moyens de resynchronisation des ventricules**
Implantierbare aktive Vorrichtung des Typs multisite mit Mitteln zum Resynchronisieren der Ventrikeln
Implantable active device of type multisite having means for resynchronization of ventricles

(30) Priorité: 17.12.1999 FR 9916015
(43) Date de publication de la demande: 20.06.2001
(73) Titulaire: ELA MEDICAL (Société anonyme), 92541 Montrouge (FR)
(72) Inventeur: Dal Molin, Renzo, 92320 Chatillon (FR); Ripart, Alain, 91190 Gif sur Yvette (FR)
(74) Mandataire: Dupuis-Latour, Dominique

(56) Documents cités:
- EP-A- 0 862 927
- WO-A-99/30777

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Elle concerne plus particulièrement les prothèses dites "multisite" dans lesquelles des électrodes sont placées en une pluralité de sites respectifs distincts comportant au moins un site ventriculaire et un site auriculaire. Il peut s'agir d'une prothèse de type "double chambre" (stimulation atriale droite et stimulation ventriculaire droite) ou, le plus souvent, "triple chambre" (stimulation atriale droite et double stimulation ventriculaire) ou "quadruple chambre" (double stimulation atriale et double stimulation ventriculaire).

En effet, outre le traitement des troubles du rythme cardiaque, il a été proposé de traiter par stimulation les troubles de contraction myocardique observés chez des patients en insuffisance cardiaque, ces troubles pouvant être spontanés ou induits par une stimulation traditionnelle. On pourra notamment se référer à l'étude de J. C. Daubert et coll., *Stimucoeur,* 25, n°3, pp. 170-176 qui fait le point des travaux sur ce sujet.

Il a été notamment proposé de stimuler simultanément les ventricules gauche et droit, en permanence, pour les resynchroniser, ce qui a permis d'observer des résultats souvent spectaculaires pour des patients en insuffisance cardiaque en classe III, non améliorée par les traitements classiques.

En pratique, ces dispositifs opèrent en stimulation synchrone, c'est-à-dire que les deux sites ventriculaires de stimulation reçoivent au même moment l'impulsion de dépolarisation (délai interventriculaire nul).

Il a été proposé (WO-A-99/30777) de faire varier le délai interventriculaire, en asservissant ce délai dans le sens de la maximisation du débit cardiaque.

Le point de départ de l'invention réside dans la constatation que cette stimulation biventriculaire à délai interventriculaire nul n'est pas nécessairement optimale, et n'aboutit pas nécessairement à une contraction synchrone des deux ventricules.

En effet, les délais de conduction au sein du myocarde ne sont pas les mêmes à droite et à gauche et peuvent dépendre de multiples facteurs, ainsi que de l'emplacement de la sonde ventriculaire gauche, selon que celle-ci est une sonde enfilée dans le sinus coronaire ou une sonde épicardique.

Il a été proposé (WO-A-99/30777) de faire varier le délai interventriculaire, en asservissant ce délai dans le sens de la maximisation du débit cardiaque.

L'un des buts de l'invention est de proposer un dispositif permettant d'établir un délai interventriculaire entre les deux stimulations, et d'ajuster ce délai de manière à resynchroniser la contraction des ventricules et aboutir ainsi à une optimisation fine de l'hémodynamique.

Ce dispositif est, de manière en elle-même connue un dispositif du type multisite tel qu'enseigné par le WO-A-99/30777 précité c'est-à-dire dans lequel des électrodes sont placées en au moins deux sites, droit et gauche, ces électrodes étant reliées à un circuit de recueil de signaux cardiaques, pour détecter un potentiel de dépolarisation, ainsi qu'à un circuit de stimulation, pour appliquer des impulsions de stimulation sur au moins certains desdits sites. Le dispositif comporte des moyens de resynchronisation des contractions ventriculaires, avec des moyens pour établir un délai entre les instants d'application des impulsions de stimulation respectives des ventricules droit et gauche, et des moyens pour faire varier ce délai.

Selon l'invention, les moyens de resynchronisation des contractions ventriculaires comportent des moyens pour analyser un paramètre représentatif du degré de désynchronisation entre ventricules sur un cycle cardiaque, et les moyens pour établir un délai entre les instants d'application des impulsions de stimulation respectives des ventricules droit et gauche font varier le délai dans le sens de la diminution du degré de désynchronisation.

Selon diverses caractéristiques subsidiaires avantageuses :
- les moyens pour analyser le paramètre représentatif comportent un capteur hémodynamique, en particulier un capteur de mesure de bioimpédance dynamique intracardiaque entre un site droit et un site gauche, notamment entre un site ventriculaire droit et un site ventriculaire gauche, ou bien entre un site atrial droit et un site ventriculaire gauche ;
- la mesure d'impédance est opérée par injection d'un courant entre un site droit et un site gauche, et recueil d'un potentiel différentiel entre un site droit et un site gauche, ou entre deux sites droits, ou entre deux sites gauches ;
- la configuration de mesure peut être une configuration quadripolaire, sans site commun à l'injection et au recueil, ou bien une configuration tripolaire, avec un site commun à l'injection et au recueil, notamment une configuration où le site commun à l'injection et au recueil est un site distal ventriculaire gauche, l'autre site d'injection est un site distal ventriculaire droit et l'autre site de recueil est un site proximal ventriculaire droit.

On va maintenant décrire un exemple de réalisation de l'invention, en référence aux dessins annexés.
La figure 1 est une vue schématique d'un muscle cardiaque avec différents sites de stimulation.
La figure 2 montre la variation d'impédance intracardiaque en fonction du temps, pendant la durée d'un cycle cardiaque.

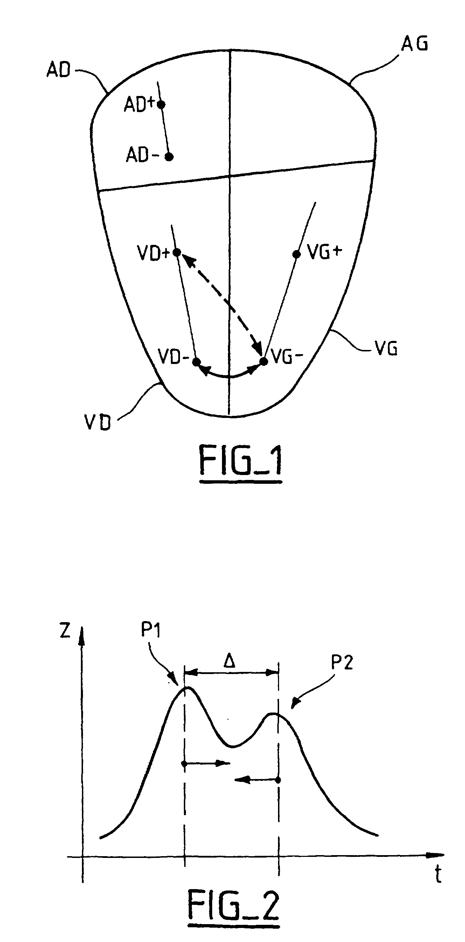

La figure 1 représente schématiquement un muscle cardiaque, avec ses quatre cavités : oreillette droite AD, oreillette gauche AG, ventricule droit VD et ventricule gauche VG.

Pour permettre une stimulation biventriculaire, des sondes ont été implantées pour la stimulation de chacun des ventricules par exemple, comme illustré, des sondes bipolaires comportant une électrode proximale ventriculaire droite VD+ une électrode distale ventriculaire droite VD-, une électrode proximale ventriculaire gauche VG+ et une électrode distale ventriculaire gauche VG-.

Une sonde peut être également implantée pour la stimulation de l'oreillette droite avec par exemple, comme illustré, une électrode proximale auriculaire droite AD+ et une électrode distale auriculaire droite AD-.

Le cas échéant, il peut être également prévu une sonde sur l'oreillette gauche, si l'on souhaite réaliser un recueil de signaux et/ou une stimulation sur les deux oreillettes (configuration quadruple chambre).

Les électrodes des sondes sont reliées à un boîtier comprenant divers circuits de détection, de stimulation et de commande, par exemple un boîtier de stimulateur multisite tel que celui décrit dans le EP-A-0 925 806 (ELA Médical) auquel on pourra se référer pour de plus amples détails.

L'introduction de délais interventriculaires de stimulation et leur ajustement pourront être réalisés par une programmation appropriée du microprocesseur du dispositif pour déclencher les différentes stimulations aux instants appropriés, ou par de la logique câblée, ou par une combinaison de moyens matériels et logiciels.

Le délai interventriculaire pourra être :
- nul,
- positif, le ventricule gauche étant stimulé après le ventricule droit avec un délai pouvant atteindre par exemple 48 ms, ou
- négatif, le ventricule droit étant stimulé après le ventricule gauche avec un délai pouvant atteindre par exemple 48 ms.

Le dispositif comporte en outre un capteur permettant de délivrer au microprocesseur un indicateur du degré de synchronisation des contractions des ventricules droit et gauche.

Avantageusement, ce dispositif est un capteur hémodynamique par mesure de bioimpédance au moyen d'un circuit tel que celui décrit par exemple dans le US-A-5 154 171 (Chirife).

Ce document décrit la manière de mesurer les volumes diastolique et systolique d'un ventricule par mesure dynamique de bioimpédance, permettant ainsi d'obtenir une indication du débit cardiaque et donc de la fraction d'éjection, qui est le paramètre hémodynamique de référence.

L'invention propose d'utiliser un circuit tel que celui décrit dans ce document mais, à la différence de celui-ci, en effectuant une mesure intracardiaque entre un site droit et un site gauche, d'une part.

La mesure de bioimpédance se fait classiquement par injection d'une impulsion de courant entre deux points, et recueil d'un potentiel différentiel entre deux points.

Dans le document précité US-A-5 154 171, les points d'injection et de recueil sont les mêmes (configuration bipolaire), tandis que dans la présente invention la configuration de mesure est une configuration tripolaire (un point commun à l'injection et au recueil) ou quadripolaire (aucun point commun à l'injection et au recueil).

Le courant injecté pour la mesure d'impédance est par exemple un courant de 80 µA délivré sous la forme d'une impulsion de largeur 5 µs.

Diverses configurations d'injection et de recueil sont possibles.

Dans une configuration actuellement préférée, qui est une configuration tripolaire, l'injection se fait (comme illustré par les flèches en trait continu épais) entre l'électrode distale ventriculaire droite VD- et l'électrode distale ventriculaire gauche VG-, le recueil étant opéré (comme illustré par les flèches en traits interrompus épais) entre l'électrode proximale ventriculaire droit VD+ et l'électrode distale ventriculaire gauche VG-.

Toutes autres configurations tripolaires ou quadripolaires sont envisageables en combinant deux à deux les six sites illustrés VD+, VD-, VG+, VG-, AD+ et AD-, les seules combinaisons exclues étant les combinaisons aboutissant à une configuration bipolaire, ou bien celles où l'injection de courant se fait d'un même côté du coeur (une injection devant être toujours réalisée entre un site droit et un site gauche).

Les configurations (tripolaires) actuellement préférées sont :
- injection entre VD- et VG-, recueil entre VD+ et VG-,
- injection entre AD- et VG-, recueil entre AD+ et VG-, et
- injection entre AD- et AG-, recueil entre AD+ et AD-.

L'injection et le recueil des signaux en ces différents points est réalisé par un circuit tel que décrit dans le US-A-5 154 171 précité, ou encore par un circuit tel que celui servant à la mesure de la ventilation-minute (MV) sur les dispositifs existants.

Dans ce dernier cas, les sites sont modifiés (injection/recueil intracardiaques, et non transpulmonaires entre coeur et boîtier), et le recueil est opéré dans des bandes de fréquences différentes : basse fréquence pour la mesure de MV, fréquence plus élevée pour la resynchronisation interventriculaire selon l'invention.

Ainsi, la partie matérielle des modules de mesure d'impédance pourra être identique à ce qui existe déjà dans les stimulateurs connus, permettant donc une mise en oeuvre de l'invention sans surcoût important.

La mesure de la ventilation-minute peut ainsi se faire par injection entre l'électrode distale ventriculaire droite VD- et le boîtier du stimulateur, et le recueil entre l'électrode proximale ventriculaire droite VD+ et le boîtier.

Sur un cycle cardiaque, la courbe d'impédance dynamique Z obtenue en fonction du temps t correspond à ce qui est illustré figure 2.

Si la contraction des ventricules n'est pas synchrone, on observe deux pics d'impédance P1 et P2 correspondant aux systoles respectives des deux ventricules, l'écart Δ entre ces deux pics étant représentatif du degré de désynchronisation interventriculaire.

Le dispositif va alors modifier l'ajustement du délai d'application des impulsions de stimulation au ventricule dans le sens de l'amélioration du synchronisme, c'est-à-dire dans le sens du rapprochement des deux pics P1 et P2, idéalement confondus en un pic unique lorsque les ventricules se contractent en synchronisme.

## Revendications

1. Un dispositif médical implantable actif, notamment un stimulateur cardiaque, défibrillateur et/ou cardioverteur, du type multisite dans lequel des électrodes sont placées en au moins deux sites, droit et gauche, ces électrodes étant reliées à un circuit de recueil de signaux cardiaques, pour détecter un potentiel de dépolarisation, ainsi qu'à un circuit de stimulation, pour appliquer des impulsions de stimulation sur au moins certains desdits sites,
ce dispositif comportant des moyens de resynchronisation des contractions ventriculaires, avec :
- des moyens pour établir un délai entre les instants d'application des impulsions de stimulation respectives des ventricules droit (VD) et gauche (VG), et
- des moyens pour faire varier ledit délai,
dispositif **caractérisé en ce que** :
- les moyens de resynchronisation des contractions ventriculaires comportent des moyens pour analyser un paramètre (Z) représentatif du degré de désynchronisation (Δ) entre ventricules sur un cycle cardiaque, et
- les moyens pour établir un délai entre les instants d'application des impulsions de stimulation respectives des ventricules droit et gauche font varier le délai dans le sens de la diminution dudit degré de désynchronisation (Δ).

2. Le dispositif de la revendication 1, dans lequel les moyens pour analyser le paramètre représentatif comportent un capteur hémodynamique.

3. Le dispositif de la revendication 2, dans lequel le capteur hémodynamique est un capteur de mesure de bioimpédance dynamique intracardiaque (Z) entre un site droit et un site gauche.

4. Le dispositif de la revendication 3, dans lequel le capteur hémodynamique est un capteur de mesure de bioimpédance dynamique (Z) entre un site ventriculaire droit (VD+, VD-) et un site ventriculaire gauche (VG+, VG-).

5. Le dispositif de la revendication 3, dans lequel le capteur hémodynamique est un capteur de mesure de bioimpédance dynamique (Z) entre un site atrial droit (AD+, AD-) et un site ventriculaire gauche (VG+, VG-).

6. Le dispositif de la revendication 3, comprenant des moyens de mesure d'impédance opérant par injection d'un courant entre un site droit (VD+, VD-, AD+, AD-) et un site gauche (VG+, VG-), et recueil d'un potentiel différentiel entre un site droit et un site gauche, ou entre deux sites droits, ou entre deux sites gauches.

7. Le dispositif de la revendication 6, dans lequel les moyens de mesure comportent une configuration tripolaire, avec un site commun à l'injection et au recueil.

8. Le dispositif de la revendication 7, dans lequel le site commun à l'injection et au recueil est un site distal ventriculaire gauche (VD-), l'autre site d'injection est un site distal ventriculaire droit (VG-) et l'autre site de recueil est un site proximal ventriculaire droit (VD+).

9. Le dispositif de la revendication 6, dans lequel les moyens de mesure comportent une configuration quadripolaire, sans site commun à l'injection et au recueil.

## Patentansprüche

1. Aktive implantierbare medizinische Vorrichtung, insbesondere ein Herzschrittmacher, Defibrillator und/oder Cardioverter, vom Mehrstellen-Typ, in welchem die Elektroden an mindestens zwei Stellen platziert sind, rechts und links, wobei die Elektroden mit einer Schaltung zum Empfang von Herzsignalen verbunden sind, um ein Depolarisations-Potential zu detektieren, sowie mit einer Stimulationsschaltung, um Stimulationsimpulse an mindestens bestimmte der genannten Stellen abzugeben,
wobei die Vorrichtung Mittel zur Resynchronisation der ventrikulären Kontraktionen umfasst, mit:
- Mitteln zum Herstellen einer Verzögerung zwischen den Zeitpunkten der Abgabe von jeweiligen Impulsen zur Stimulation des rechten (VD) und linken (VG) Ventrikels, und
- Mitteln zum Variieren der genannten Verzögerung,
wobei die Vorrichtung **dadurch gekennzeichnet ist, dass**:
- die Mittel zur Resynchronisation der ventrikulären Kontraktionen Mittel zum Analysieren eines Parameters (Z) aufweisen, der repräsentativ für den Grad der Desynchronisation (Δ) zwischen den Ventrikeln über einen Herzzyklus ist, und
- die Mittel zum Herstellen einer Vezögerung zwischen den Zeitpunkten der Abgabe von jeweiligen Impulsen zur Stimulation der rechten und linken Ventrikel, die Verzögerung in Richtung der Verminderung des genannten Desynchronisationsgrades (Δ) variieren.

2. Vorrichtung nach Anspruch 1, in welcher die Mittel zur Analyse des repräsentativen Parameters einen hämodynamischen Sensor aufweisen.

3. Vorrichtung nach Anspruch 2, in welcher der hämodynamische Sensor ein Sensor zur Messung der intrakardischen dynamischen Bioimpedanz (Z) zwischen einer rechten Stelle und einer linken Stelle ist.

4. Vorrichtung nach Anspruch 3, in welcher der hämodynamische Sensor ein Sensor zur Messung der dynamischen Bioimpedanz (Z) zwischen einer rechten ventrikulären Stelle (VD+, VD-) und einer linken ventrikulären Stelle (VG+, VG-) ist.

5. Vorrichtung nach Anspruch 3, in welcher der hämodynamische Sensor ein Sensor zur Messung der dynamischen Bioimpedanz (Z) zwischen einer rechten atrialen Stelle (AD+, AD-) und einer linken ventrikulären Stelle (VG+, VG-) ist.

6. Vorrichtung nach Anspruch 3, die Mittel zur Messung der Impedanz aufweist, welche durch Injektion eines Stroms zwischen einer rechten Stelle (VD+, VD-, AD+, AD-) und einer linken Stelle (VG+, VG-) arbeiten, und durch Empfang eines differentiellen Potentials zwischen einer rechten Stelle und einer linken Stelle, oder zwischen zwei rechten Stellen, oder zwischen zwei linken Stellen.

7. Vorrichtung nach Anspruch 6, in welcher die Mittel zur Messung eine tripolare Konfiguration umfassen, mit einer gemeinsamen Stelle zur Injektion und zum Empfang.

8. Vorrichtung nach Anspruch 7, in welcher die gemeinsame Stelle zur Injektion und zum Empfang eine linke ventrikuläre distale Stelle (VD-) ist, die andere Stelle zur Injektion eine rechte ventrikuläre distale Stelle (VG-) ist und die andere Stelle zum Empfang eine rechte ventrikuläre proximale Stelle (VD+) ist.

9. Vorrichtung nach Anspruch 6, in welcher die Mittel zur Messung eine quadripolare Konfiguration umfassen, ohne gemeinsarne Stelle zur Injektion und zum Empfang.

## Claims

1. An active implantable medical device, in particular a cardiac pacemaker, defibrillator and/or cardioverter, of the multisite type wherein electrodes are placed in at least two sites, right and left, said electrodes being connected to a cardiac signal sensing circuit for detecting a depolarisation potential, as well as to a stimulation circuit for applying stimulation pulses to at least some of said sites.
said device comprising means for resynchronisation of ventricular contractions, including:
- means for establishing a delay between the times of application of respective stimulations pulse to right (RV) and left (LV) ventricles, and
- means for varying said delay,
said device being **characterised in that**:
- the means for resynchronisation of ventricular contractions includes means for analysing a parameter (Z) representative of the degree of desynchronisation (Δ) between the ventricles over a cardiac cycle, and
- the means for establishing a delay between the times of application of respective stimulations pulse to right and left ventricles vary the delay in the direction which reduces said degree of desynchronisation (Δ).

2. The device of claim 1, wherein the means for analysing the representative parameter comprises a hemodynamic sensor.

3. The device of claim 2, wherein the hemodynamic sensor is a sensor for measuring an intracardiac dynamic bioimpedance (Z) between a left site and a right site.

4. The device of claim 3, wherein the hemodynamic sensor is a sensor for measuring an intracardiac dynamic bioimpedance (Z) between a right ventricular site (VD+, VD-) and a left ventricular site (VG+, VG-).

5. The device of claim 3, wherein the hemodynamic sensor is a sensor for measuring an intracardiac dynamic bioimpedance (Z) between a right atrial site (AD+, AD-) and a left ventricular site (VG+, VG-).

6. The device of claim 3, including impedance measurement means which operates through injection of a current between a right site (VD+, VD-, AD+, AD-) and a left site (VG+, VG-), and sensing of a differential potential between a right site and a left site, or between two right sites, or between two left sites.

7. The device of claim 6, wherein the measurement means includes a tripolar configuration, with one site common to injection and sensing.
the collection of the differential potential. The configuration of the bioimpedance measurement can be a configuration, without a site common to the injection and the collection, or a tripolar configuration, with one site common to the injection and the collection

8. The device of claim 7, wherein the site common to injection and sensing is a left ventricular distal site (VD-), the other injection site is a right distal ventricular site (VG-), and the other sensing site is a right proximal ventricular site (VD+).

9. The device of claim 6, wherein the measurement means includes a quadripolar configuration, with no site common to injection and sensing.
